# EUROPÄISCHE PATENTSCHRIFT

(11) **EP 0 401 474 B1**
(45) Veröffentlichungstag und Bekanntmachung des Hinweises auf die Patenterteilung: **12.10.1994**
(21) Anmeldenummer: 90104497.4
(22) Anmeldetag: 09.03.1990
(51) Int. Cl.: A61B 17/22

(54) **Kinematik eines Lithotripters**
Kinematics of a lithotripter
Cinématique d'un lithotripteur

(30) Priorität: 11.05.1989 DE 3915383
(43) Veröffentlichungstag der Anmeldung: 12.12.1990
(73) Patentinhaber: DORNIER MEDIZINTECHNIK GMBH, D-82101 Germering (DE)
(72) Erfinder: Viebach, Thomas, Dipl.-Ing., D-8081 Pischertshofen (DE); Buchbauer, Peter, Dipl.-Ing., D-8046 Garching (DE)
(74) Vertreter: Landsmann, Ralf, Dipl.-Ing.

(56) Entgegenhaltungen:
- EP-A- 0 265 742
- EP-A- 0 286 170
- DE-U- 8 804 420

## Beschreibung

Die Erfindung betrifft eine Vorrichtung zur Behandlung von Patienten mit fokussierten Stosswellen, insbesondere einen Lithotripter mit einem Therapiekopf, der Mittel zum Erzeugen, Fokussieren und Einleiten von Stosswellen in einen Patientenkörper enthält.

Es sind Geräte für die berührungsfreie Steinzerkleinerung bekannt, bei denen der Stosswellenerzeuger ortsfest angebracht ist und der Patient auf einer Liege so verschoben wird, daß der zu behandelnde Stein in den Brennpunkt des Stosswellensystems verschoben wird. Bekannt sind ebenfalls Lithotriptoren, bei denen außer der Patientenliege auch das Stosswellensystem (das sich dann in einem Therapiekopf befindet, in dem gleichzeitig Mittel zum Fokussieren und Einleiten der Stosswellen in den Körper angeordnet sind) beweglich ist. Mit diesen Geräten ist die Positionierung des Patienten vereinfacht.

Aus der EP-A-0 286 170 ist ein Lithotripsie-Arbeitsplatz mit Patientenlagerungstisch zur Behandlung von Patienten mit fokussierten Stosswellen bekannt, der einen Therapiekopf, Mittel zum Erzeugen der Stosswellen und zum Einleiten in den Patientenkörper aufweist, wobei die Achse des Therapiekopfes auf einem Kegelmantel beweglich ist.

Aufgabe der Erfindung ist es, eine Lithotripsievorrichtung der bekannten Art bezüglich der Ortung und der Positionierung des Patienten zu vereinfachen

Diese Aufgabe wird erfindungsgemäß gelöst von einer Vorrichtung mit den Merkmalen des Anspruchs 1. Ausführungen der Erfindung sind Gegenstände von abhängigen Ansprüchen.

Erfindungsgemäß wird der Therapiekopf so angeordnet, daß er durch eine Zwangsführung auf einem Kegelmantel beweglich ist, wobei die Spitze des Kegelmantels mit dem Fokus des Therapiekopfes zusammenfällt (isozentrische Bewegung). Der Kegelmantel liegt bevorzugt so, daß eine seiner Erzeugenden senkrecht verläuft, das heißt, daß der Therapiekopf auch in eine Stellung gebracht werden kann, in der seine Längsachse senkrecht verläuft.

In einer Ausführung kann eine Zwangsführung vorgesehen sein, die den Therapiekopf beim Verschwenken auf dem Kegelmantel so verdreht, daß er bezüglich des Gesamtgeräts keine Verdrehung um seine Längsachse durchführt. Damit werden unbeabsichtigte Verschiebungen bei Ortungsaufnahmen verhindert.

In einer Ausführung ist der Therapiekopf an zwei zweiarmigen Hebeln angelenkt, wobei der erste Arm im wesentlichen horizontal verläuft und an seinem Ende ein Lager für den zweiten Arm ausgebildet ist, dessen Drehachse mit der Senkrechten einen Winkel α , der zwischen 0° und 45° liegt, einschließt. In bevorzugten Ausführungen liegt der Winkel α zwischen 10 und 30°, insbesondere bei 15°. Wenn der Winkel zwischen der Hauptachse des Therapiekopfes und der vorgenannten Drehachse zwischen den beiden Armen ebenfalls den Wert des Winkel α aufweist würde dann ein Kegel mit einer Öffnung von insgesamt 30° entstehen und wenigstens eine Erzeugende des Kegels senkrecht stehen.

In einer Ausführung kann der gesamte Therapiekopf mit seiner Mechanik um eine senkrechtstehende Achse in eine Parkposition verschiebbar sein. Dies dann z.B. dadurch erreicht werden, daß der erste Arm um eine senkrechte Achse drehbar ist. Damit kann der Therapiekopf aus dem Arbeitsbereich des Arztes verschwenkt werden, so daß am Lithotripter auch andere medizinische Maßnahmen (z.B. endourologische oder transurethrale Maßnahmen) durchgeführt werden können.

In einer Ausführung ist am Therapiekopf ein Ultraschall-Ortungsgerät so befestigt, daß dessen Mittelachse stets auf den Brennpunkt des Therapiekopfes zielt. Dies kann insbesondere durch eine Zwangsführung erfolgen und hat den Vorteil, daß auf dem Ultraschallbild in der Mittellinie stets der Fokus liegt, wodurch die Ortung wesentlich vereinfacht wird. Der Arzt hat eine relativ große Freiheit, Fenster für das Ultraschallbild am Patientenkörper zu suchen und verliert dabei nie den Fokus aus der Bildmitte.

Der Ultraschall-Transducer kann z.B. an einem Ring drehbar am Therapiekopf befestigt sein, so daß die Drehbewegung um die Längsachse des Therapiekopfes erfolgt. Die isozentrische Bewegung des Transducers kann von einer Kinematik erzwungen werden, die drei Hebel enthält, wobei der erste und der dritte Hebel so geführt sind, daß sie stets parallel verlaufen. Möglich sind Ausführungen, bei denen der Transducer in seiner Längsachse verschieblich ist, wodurch er sich den unterschiedlichen Abständen von der Patientenoberfläche zum Stein anpassen kann, oder bei denen der Transducer um seine eigene Längsachse drehbar ist, so daß die Suche mit Ultraschall nochmals vereinfacht wird.

Die Erfindung wird anhand zweier Figuren näher erläutert.

### Es zeigen:

- Figur 1: drei Ansichten eines erfindungsgemäßen Lithotripters,
- Figur 2: die Bewegungsmöglichkeiten des Therapiekopfes und des Transducers einer Ausführung.

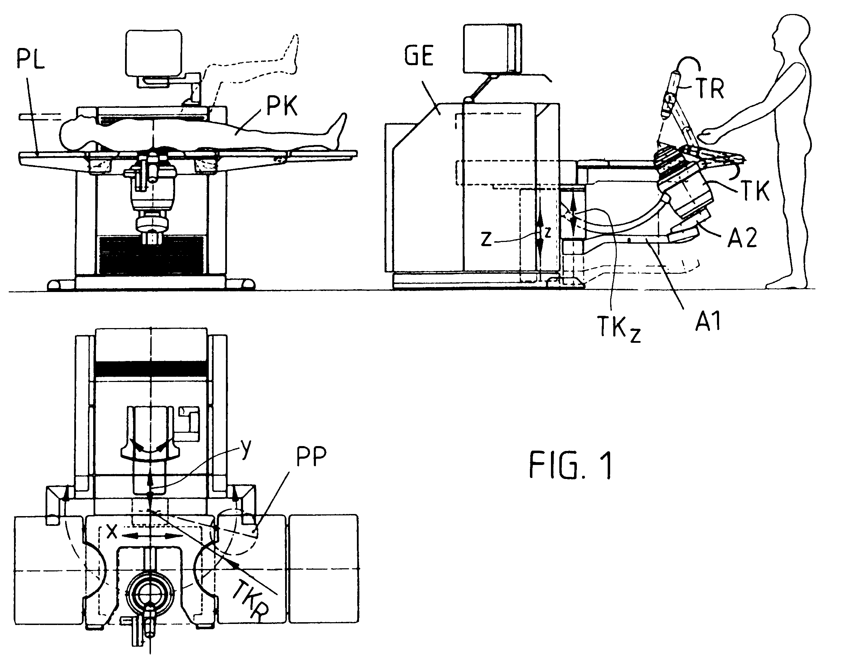

Figur 1 zeigt einen Lithotripter, bei dem am Geräterahmen GE eine patientenliege PL auskragend befestigt ist. Der Therapiekopf, der eine Stosswellenquelle, Mittel zum Fokussieren und Mittel zum Einleiten der Stosswelle in den Patientenkörper PK enthält, ist hier an zwei Armen A1, A2 befestigt. Am Therapiekopf TK befindet sich zwangsgeführt der Transducer TR. In Figur 1 sind die Bewegungsmöglichkeiten der Liege in z-Richtung und des Therapiekopfes in z-Richtung TK_{z} gezeigt. Die Liege kann horizontal in x- und y-Richtung (in Richtung der Patientenlängsachse und senkrecht dazu) bewegt werden. Der Therapiekopf kann, wie Figur 1 unten zeigt, durch Verschwenken um eine senkrechte Achse in eine Parkposition PP verschwenkt werden, so daß der Raum unter der Patientenliege PL frei zugänglich ist. Durch die Verschiebung der Patientenliege PL in z-Richtung kann die Höhe der Liege frei eingestellt werden. Gezeigt sind z.B. die Aufstiegshöhe A, eine individuelle Behandlungshöhe B und die Steinschnittlagenhöhe C.

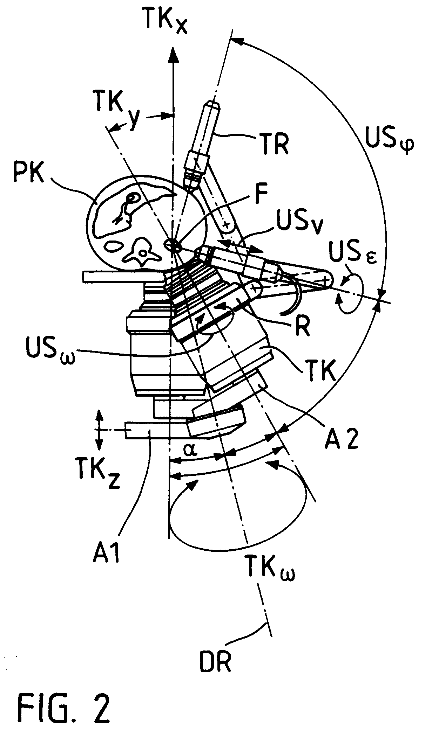

Figur 2 zeigt die Bewegungsmöglichkeiten eines erfindungsgemäßen Therapiekopfes TK. Die Längsachse des Therapiekopfes TK kann sich auf einem Kegel bewegen, dessen Spitze durch den Brennpunkt F des Stosswellen-Fokussierungssystems vorgegeben ist. Dieser Brennpunkt F fällt zugleich mit dem Isozentrum des beweglichen Transducers TR zusammen. Der Therapiekopf ist hier an den Armen A1 und A2 befestigt. Der Arm A2 rotiert um die Drehachse DR, wodurch die Bewegung des Therapiekopfes TK auf dem Kegelmantel erzeugt wird. Die Drehachse DR ist um den Winkel α , hier 15°, gegen die Senkrechte verkippt. Auch die Längsachse des Therapiekopfes TK ist um den gleichen Winkel gegen die Drehachse DR verkippt. In der Figur sind folgende Bewegungsmöglichkeiten gezeigt:
- TK_{z}:: Bewegung des Therapiekopfes in z-Richtung (auf und ab)
- TK_{ω}:: Bewegung des Therapiekopfes TK auf einem Kegelmantel.

Damit ergibt sich die Verschwenkungsmöglichkeit des Therapiekopfes gegenüber der x- und y-Richtung (TKₓ, TK_{y}) zu je 30°.

Am Therapiekopf TK ist der Ultraschall-Transducer TR zwangsgeführt. Er ist hier mit dem Ring R und drei zweiarmigen Hebeln am Therapiekopf befestigt. Seine Bewegungsmöglichkeiten sind:
- US_{ω}:: Drehung um die Therapiekopf-Hauptachse,
- US_{φ}:: Verschwenkung des Transducers TR um eine Achse, die senkrecht zur Längsachse des Therapiekopfes verläuft,
- US_{ε} :: Drehung um die Längsachse des Transducers,
- USᵥ :: Bewegung in Richtung der Längsachse des Transducers.

Die Bewegung des Transducers TR in Längsrichtung (USᵥ) wird bevorzugt elektronisch aufgenommen und die Lage des therapeutischen Fokus dann im Ultraschallbild angezeigt.

Durch die gezeigten Bewegungsmöglichkeiten des Therapiekopfes TK und des Ultraschall-Beobachtungsgerätes TR ist der Arzt relativ frei in der Wahl der Beobachtungsfenster und der Behandlungsfenster.

## Patentansprüche

1. Vorrichtung zur Behandlung von Patienten mit fokussierten Stosswellen, insbesondere Lithotripter, mit einem Therapiekopf (TK), der Mittel zum Erzeugen, Fokussieren und Einleiten von Stosswellen in einen Patientenkörper (PK) enthält, wobei die Achse des Therapiekopfes (TK) auf einem Kegelmantel beweglich ist, dessen Spitze im Fokus (F) des Therapiekopfes (TK) liegt (Isozentrum), **dadurch gekennzeichnet**, daß der Therapiekopf (TK) an einem zweiarmigen Hebel (Arm A1, Arm A2) angelenkt ist, wobei der erste Arm (A1) im wesentlichen horizontal verläuft und an seinem Ende ein Lager für den zweiten Arm (Arm 2) aufweist, dessen Drehachse (DR) mit der Senkrechten einen Winkel α einschließt, der zwischen 0 und 45°, bevorzugt zwischen 10 und 30°, insbesondere bei 15° liegt.

2. Vorrichtung nach Anspruch 1, **dadurch gekennzeichnet**, daß der Therapiekopf (TK) durch eine Zwangsführung beim Verschwenken auf dem Kegelmantel so verdreht wird, daß er bezuglich des Gesamtgeräts (GE) keine Verdrehung um seine Längsachse durchführt.

3. Vorrichtung nach Anspruch 1, **dadurch gekennzeichnet**, daß die Hauptachse des Therapiekopfes (TK) mit der Drehachse (DR) zwischen beiden Armen (A1, A2) den gleichen Winkel α einschließt.

4. Vorrichtung nach Anspruch 1 oder 3, **dadurch gekennzeichnet**, daß der erste Arm (A1) an seinem Anfang um eine senkrechte Achse drehbar an dem Gehäuse (GE) des Lithotripters befestigt ist.

5. Vorrichtung nach einem der vorhergehenden Ansprüche, **dadurch gekennzeichnet**, daß alle Bewegungsmöglichkeiten um Achsen in Bezug auf die Erdoberfläche mit Bremsen versehen sind.

6. Vorrichtung nach einem der vorhergehenden Ansprüche, **dadurch gekennzeichnet**, daß am Therapiekopf (TK) ein Ultraschall-Transducer (TR) befestigt ist, der so geführt ist, daß seine Hauptachse auf den Fokus (F) des Therapiekopfes (TK) gerichtet ist.

7. Vorrichtung nach einem der vorhergehenden Ansprüche, **dadurch gekennzeichnet**, daß der Transducer (TR) an einem den Therapiekopf (TK) umfassenden Ring (R) befestigt ist, dessen Achse mit der Achse des Therapiekopfes (TK) zusammenfällt.

8. Vorrichtung nach einem der vorhergehenden Ansprüche, **dadurch gekennzeichnet**, daß der Transducer (TR) mit einer Kinematik am Therapiekopf (TK) befestigt ist, die drei Hebel enthält, wobei der erste und der dritte Hebel so zwangsgeführt sind, daß sie parallel zueinander verlaufen.

9. Vorrichtung nach einem der vorhergehenden Ansprüche, **dadurch gekennzeichnet**, daß der Transducer (TR) in seiner Längsachse verschieblich ist.

10. Vorrichtung nach einem der vorhergehenden Ansprüche, **dadurch gekennzeichnet**, daß der Transducer (TR) um seine Längsachse drehbar ist.

## Claims

1. Device for the treatment of patients with focussed shockwaves, in particular a lithotripter, comprising a therapy head (TK) Including means for producing, focussing and introducing shockwaves in the body of a patient (PK), in which respect the axis of the therapy head (TK) is movable on a conical casing the tip of which is in the focus of the therapy head (TK)(iso-centre), **characterised in that** the therapy head (TK) is hinged onto a two-armed lever (Arm A1, Arm A2), and that the first arm (A1) extends essentially horizontally and has at its end a mount for the second arm (Arm 2), the rotary axis (DR) of which and the vertical include an angle α between 0 and 45°, preferably between 10 and 30°, in particular at 15°.

2. Device according to claim 1, **characterised in that** the therapy head (TK) is rotated by forced guidance on the conical casing whilst being pivoted in such a manner that it does not perform a rotation around its longitudinal axis relative to the entire device (GE).

3. Device according to claim 1, **characterised in that** the main axis of the therapy head (TK) and the rotary axis (DR) include the same angle α between both arms (A1, A2).

4. Device according to claim 1 or 3, **characterised in that** the first arm (A1) is at its start mounted to the housing (GE) of the lithotripter so as to be rotatable around a vertical axis.

5. Device according to one of the abaove claims, **characterised in that** all possibilities of movement around axes relative to the earth surface are provided with brakes.

6. Device according to one of the above claims, **charactarised in that** attached to the therapy head (TK) is an ultrasound transducer (TR) which is guided in such a manner that its main axis is oriented towards the focus (F) of the therapy head (TK).

7. Device according to one of the above claims, **characterised in that** the transducer (TR) is mounted on a ring (R) which spans around the therapy head (TK) and the axis of which coincides with the axis of the therapy head (TK).

8. Device according to one of the above claims, **characterised in that** the transducer is mounted on the therapy head (TK) with a cinematic which has three levers, and that the first and third lever or forcibly guided in such a manner that they extend parallel to each other.

9. Device according to one of the above claims, **characterised in that** the transducer (TR) is displaceable in its longitudinal axis.

10. Device according to one of the above claims, **characterised in that** the transducer (TR) is rotatable around its longitudinal axis.

## Revendications

1. Dispositif pour le traitement de patients au moyen d'ondes de choc focalisées, notamment lithotriteur, comportant une tête de de traitement (TK) qui comprend des moyens pour produire, focaliser et introduire des ondes de choc dans le corps (PK) d'un patient, l'axe de la tête de traitement (TK) étant mobile suivant une enveloppe de cône dont le sommet coïncide avec le foyer (F) de la tête de traitement (TK) (isocentre), caractérisé par le fait que la tête de traitement (TK) est montée de manière articulée sur un levier à deux bras (bras A1, bras A2), le premier bras (A1) étant sensiblement horizontal et présentant a l'une de ses extrémités un palier pour le deuxième bras (A2) dont l'axe de rotation (DR) forme avec la verticale un angle α compris entre 0 et 45°, de préférence entre 10 et 30°, notamment de 15°.

2. Dispositif selon la revendication 1, caractérisé par le fait que lors de son pivotement sur l'enveloppe de cône, la tête de traitement (TK) est soumise à une rotation par un moyen de guidage de telle sorte que ladite tête ne tourne pas autour de son axe longitudinal par rapport à l'ensemble de l'appareil (GE).

3. Dispositif selon la revendication 1, caractérisé par le fait que l'axe principal de la tête de traitement (TK) forme avec l'axe de rotation (DR) entre les deux bras (A1, A2) le même angle α.

4. Dispositif selon la revendication 1 ou 3, caractérisé par le fait que le premier bras (A1), au niveau de son origine, est fixé sur la carrosserie (GE) du lithotriteur avec possibilité de rotation autour d'un axe vertical.

5. Dispositif selon l'une des revendications précédentes, caractérisé par le fait que toutes les possibilités de mouvement autour d'axes par rapport à la surface du sol sont freinées.

6. Dispositif selon l'une des revendications précédentes, caractérisé par le fait qu'un transducteur à ultrasons (TR) est fixé sur la tête de traitement (TK) et est guidé de manière telle que son axe principal soit dirigé sur le foyer (F) de la tête de traitement (TK).

7. Dispositif selon l'une des revendications précédentes, caractérisé par le fait que le transducteur est fixé sur un anneau (R) qui entoure la tête de traitement (TK) et dont l'axe coïncide avec l'axe de ladite tête de traitement (TK).

8. Dispositif selon l'une des revendications précédentes, caractérisé par le fait que le transducteur (TR) est fixé sur la tête de traitement (TK) au moyen d'un ensemble cinématique, qui comprend trois leviers, le premier et le troisième levier étant guidés de telle sorte que ceux-ci soient mutuellement parallèles.

9. Dispositif selon l'une des revendications précédentes, caractérisé par le fait que le transducteur (TR) est mobile dans la direction de son axe longitudinal.

10. Dispositif selon l'une des revendications précédentes, caractérisé par le fait que le transducteur (TR) est mobile en rotation autour de son axe longitudinal.
